# EUROPEAN PATENT SPECIFICATION

(11) **EP 2 942 077 B1**
(45) Date of publication and mention of the grant of the patent: **28.12.2016**
(21) Application number: 15162879.9
(22) Date of filing: 09.04.2015
(51) Int. Cl.: A61M 25/00

(54) **MOUNTING CARD**
BEFESTIGUNGSKARTE
CARTE DE MONTAGE

(30) Priority: 10.05.2014 JP 2014098192
(43) Date of publication of application: 11.11.2015
(73) Proprietor: ASAHI INTECC CO., LTD., Nagoya-shi, Aichi 463-0024 (JP)
(72) Inventor: Katsuno, Ryota c/o ASAHI INTECC CO., LTD., Nagoya-shi, Aichi 463-0024 (JP); Take, Rin c/o ASAHI INTECC CO., LTD., Nagoya-shi, Aichi 463-0024 (JP)
(74) Representative: Winter, Brandl, Fürniss, Hübner, Röss, Kaiser, Polte - Partnerschaft mbB

(56) References cited:
- EP-A1- 0 913 164
- JP-A- 2000 255 627
- US-A1- 2005 194 276
- US-A1- 2010 025 273

## Description

The present invention relates to a mounting card.

### [Background Art]

Conventionally, a flexible medical component such as a catheter is typically held on a mounting card having a flat surface and packaged in a sterile condition. Such a mounting card can be found in US2010/025273. Such a mounting card has a plurality of tabs for holding a flexible medical component such as a catheter. The tabs are formed by making cuts such as a V -shaped cut or a U-shaped cut on the mounting card. The tabs formed by cutting the mounting card are slightly lifted from the flat surface of the mounting card to hold the flexible medical component between the flat surface of the mounting card and the tabs. The flexible medical component is held thus on the mounting card. In this case, if a holding force of the tab, that is, a force for returning the tab to the flat surface is small, a force for holding the flexible medical component becomes insufficient. If the holding force of the tab, that is, the force for returning the tab to the flat surface is large, another problem arises such that it becomes hard to get out the packaged flexible medical component from the package when used. If the flexible medical component is an elongated flexible medical component, in particular, the proximal side of the elongated flexible medical component often be held and then the elongated flexible medical component often be pulled out from the package when the elongated flexible medical component is got out from the package. In this case, if a distal portion of the flexible medical component is not linear like a catheter having a curved distal portion, the distal portion of the flexible medical component may be caught by the tab and it may be responsible for deformation or breakage of the distal portion.

For example, Patent Literature 1 discloses a mounting card having a flat surface that holds an elongated flexible medical component, e.g., a catheter. The flat surface has relatively large tabs and relatively small tabs in pairs that hold the flexible medical component. The relatively large tabs and the relatively small tabs are formed by making triangular (U-shaped) cuts with rounded tips on the mounting card. The tabs shaped thus in pairs reduce damage of the flexible medical component when the flexible medical component is got out from the mounting card.

Moreover, for example, Patent Literature 2 discloses a mounting card that has a flat surface for holding an elongated flexible medical component, e.g., a catheter. The flat surface has pairs of L-shaped or mountain-shaped tabs that hold the flexible medical component. The L-shaped or mountain-shaped tabs are formed by making cuts on a mounting card. A pulling resistance of the flexible medical component is reduced by cuts made on both ends of the tabs toward the distal end side of the flexible medical component.

As described above, conventionally, a tab shape for a mounting card has been proposed so as to prevent damage on a flexible medical component with a small pulling resistance even in a case that the flexible medical component is pulled out.

However, flexible medical components have various shapes and a mounting card that can apply to flexible medical components varying in shape has been demanded. Furthermore, a mounting card having a sufficient holding force for holding a flexible medical component and prevent deformation or breakage on the flexible medical component even in a case that the flexible medical component is pulled out from the mounting card.

### [Citation List]

### [Patent Literature]

[Patent Literature 1] National Publication of Translated Version No. 2007-527295
[Patent Literature 2] Japanese Patent Laid-Open No. 2009-201826

### [Summary of Invention]

### [Technical Problem]

An obj ect of the present invention is to provide a mounting card as described in claim 1 having a flat surface for holding an elongated flexible medical component, the mounting card having a sufficient holding force for holding the flexible medical component and preventing deformation or breakage of the flexible medical component even if the flexible medical component is pulled out from the proximal side.

### [Solution to Problem]

The present invention is a mounting card having a flat surface for holding an elongated flexible medical component, wherein
the flat surface comprises a U-shaped distal-side tab and a proximal-side tab that are formed in a pair so as to hold the flexible medical component on the flat surface, the distal-side tab and the proximal-side tab are formed in a pair such that the distal-side tab is located closer to the distal end side of the flexible medical component than the proximal-side tab when the flexible medical component is held on the mounting card, the distal-side tab is formed by making a U-shaped cut on the mounting card such that a tip of the distal-side tab is oriented toward the distal end side of the flexible medical component, and the proximal-side tab is formed by making a cut on the mounting card such that a distal side, which is located closer to the distal end side of the flexible medical component, of the cut of the proximal-side tab is disposed in parallel with a proximal side, which is located closer to a proximal end side of the flexible medical component, of the U-shaped cut of the distal-side tab and a distance between the distal side of the cut of the proximal-side tab and the proximal side of the U-shaped cut of the distal-side tab is larger than the diameter of the flexible medical component to be held.

The proximal-side tab may be preferably formed by making a V-shaped cut on the mounting card.

### [Advantageous Effects of Invention]

The mounting card of the present invention has the flat surface having the U-shaped distal-side tab and the proximal-side tab that are formed in a pair so as to hold the flexible medical component on the flat surface, the distal-side tab is formed by making the U-shaped cut on the mounting card such that the tip of the distal-side tab is oriented toward the distal end side of the flexible medical component, and the proximal-side tab is formed by making the cut on the mounting card such that the distal side, which is located closer to the distal end side of the flexible medical component, of the cut of the proximal-side tab is disposed in parallel with the proximal side, which is located closer to the proximal end side of the flexible medical component, of the U-shaped cut of the distal-side tab and a distance between the distal side of the cut of the proximal-side tab and the proximal side of the U-shaped cut of the distal-side tab is larger than the diameter of the flexible medical component. Thus, even if the flexible medical component having a curved distal portion is held on the mounting card, the curved distal portion is not caught when the flexible medical component is pulled out from the mounting card and deformation or breakage of the flexible medical component is avoided. Furthermore, the flexible medical component is reliably held by the distal-side tab and the proximal-side tab with an excellent holding force.

If the proximal-side tab is V-shaped, the proximal-side tab has a long base on which the mounting card and the proximal-side tab are not separated from each other. This provides an excellent holding force for holding the flexible medical component without excessively increasing the size of the proximal-side tab, and a sufficient holding force for holding the flexible medical component is easily obtained.

### [Brief Description of Drawings]

[FIG. 1] FIG. 1 is a front view schematically showing an embodiment of a mounting card of the present invention.
[FIG. 2] FIG. 2(a) is an enlarged view of a part A of FIG. 1, and FIG. 2(b) is an enlarged view of a part B of FIG. 1.
[FIG. 3] FIG. 3 is a front view schematically showing a state in use in which a flexible medical component is held on the mounting card according to the embodiment of the present invention.

### [Description of Embodiment]

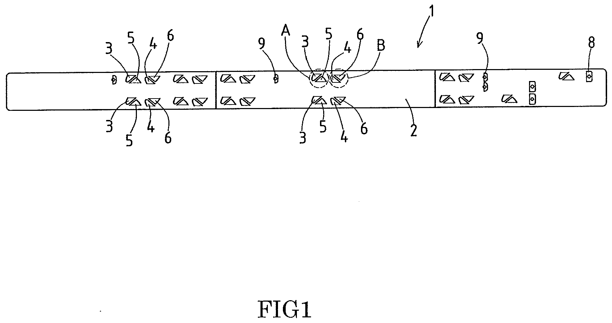

An embodiment of the present invention will be described below with reference to the accompanying drawings. The present invention is not limited to the embodiment. In FIG. 1, the left side of the drawing is a distal end side of a flexible medical component held on a mounting card, and the right side of FIG. 1 is a proximal end side of the flexible medical component held on the mounting card. A connector or the like is connected in the proximal end side of the flexible medical component.

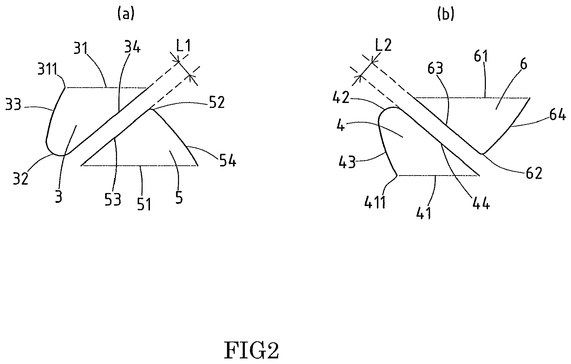

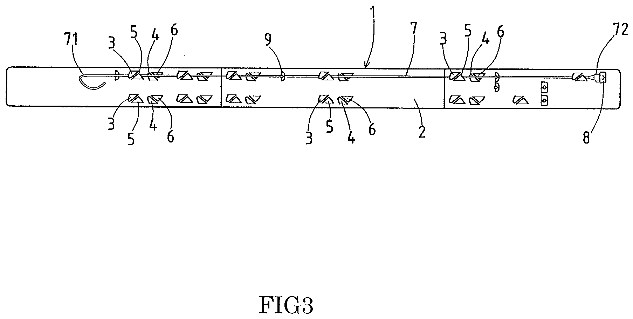

FIG. 1 is a front view schematically showing a first embodiment of the mounting card of the present invention. FIG. 2(a) is an enlarged view of a part A of FIG. 1. FIG. 2(b) is an enlarged view of a part B of FIG. 1. FIG. 3 is a front view schematically showing a state in use in which the flexible medical component is held on the mounting card according to the first embodiment of the present invention.

A mounting card 1 has a flat surface 2 on which distal-side tabs 3 and 4 and proximal-side tabs 5 and 6 are formed. The distal-side tabs 3 and 4 and the proximal-side tabs 5 and 6 are formed by making cuts on the mounting card 2 with a cutter and so on. The distal-side tabs 3 and the distal-side tabs 4 are faced toward different directions. The distal-side tab 3 has a tab base 31 that is not separated from the flat surface 2 and is located on the upper side of FIG. 2 (a). The distal-side tab 4 has a tab base 41 that is not separated from the flat surface 2 and is located on the lower side of FIG. 2(b). Similarly, the proximal-side tab 5 and the proximal-side tab 6 are faced toward different directions. The proximal-side tab 5 has a tab base 51 that is not separated from the flat surface 2 and is located on the lower side of FIG. 2 (a). The proximal-side tab 6 has a tab base 61 that is not separated from the flat surface 2 and is located on the upper side of FIG. 2(b). The distal-side tabs 3 and 4 and the proximal-side tabs 5 and 6 are formed such that the distal-side tab 3 and the proximal-side tab 5 are formed in a pair so as to hold a flexible medical component 7 while the distal-side tab 4 and the proximal-side tab 6 are formed in a pair so as to hold the flexible medical component 7. Any one of the pairs may be formed on the flat surface 2. However, both of the pairs are preferably formed on the flat surface 2 so as to make a choice between the pairs depending on the flexible medical component 7 to be held.

The shape and size of the mounting card 1 are not particularly limited as long as the elongated flexible medical component 7 can be held. For example, if the elongated flexible medical component 7 to be held is a catheter, the mounting card 1 is preferably a rectangular sheet which is longer than the catheter. The mounting card 1 is preferably sized and shaped so as to hold all of the flexible medical component 7. However, the mounting card 1 may be sized and shaped so as to hold a part of the flexible medical component 7. FIG. 3 shows a state in use in which the elongated flexible medical component 7, e.g., a catheter is held on the mounting card 1. The mounting card 1 is a rectangular sheet that can hold all of the flexible medical component 7, e.g., a catheter. The flexible medical component 7 is placed on the flat surface 2 and then is held between the flat surface 2 and the distal-side tab 3 and proximal-side tab 4.

A material forming of the mounting card 1 is not particularly limited and includes any material which is used conventionally such as paper, resin, and resin foam.

The mounting card 1 has the flat surface 2 for holding the flexible medical component 7. The flat surface 2 does not need to be precisely flat as long as the flexible medical component 7 can be placed on the mounting card 1.

The distal-side tabs 3 and 4 are U-shaped with smooth tips 32 and 42 and are located closer to the distal end side than the proximal-side tabs 5 and 6 which are arranged in pairs with the distal-side tabs 3 and 4. The U-shape includes, as shown in FIGS. 1 to 3, a shape of deformed alphabet U.

The distal-side tabs 3 and 4 are formed by making U-shaped cuts on the mounting card 1 such that the tips 32 and 42 of the distal-side tabs 3 and 4 are oriented toward the distal end side. The state in which the tips 32 and 42 are oriented toward the distal end side means that the tips 32 and 42 are located closer to the distal end side than distal ends 311 and 411 of the tab bases 31 and 41 in the axial direction of the flexible medical component 7 to be held.

The distal-side tabs 3 and 4 have distal sides 33 and 43, and the distal sides 33 and 43 may be linear or smoothly curved. The distal-side tabs 3 and 4 have proximal sides 34 and 44, and the proximal sides 34 and 44 may be linear or smoothly curved. The proximal sides 34 and 44 are preferably linear in view of the adjustability of a positional relationship with distal sides 53 and 63 of the proximal-side tabs 5 and 6, which will be described later.

Since the distal-side tabs 3 and 4 are shaped thus, even if the flexible medical component 7 having a curved distal portion 71 is held on the mounting card 1, the flexible medical component 7 can be got out from the mounting card 1 without catch of the distal portion 71 of the flexible medical component 7 by the distal-side tabs 3 and 4. Thus, the flexible medical component 7 is not deformed or broken by the distal-side tabs 3 and 4.

The proximal-side tabs 5 and 6 are located closer to the proximal end side than the distal-side tabs 3 and 4 that are paired with the proximal-side tabs 5 and 6, respectively.

The proximal-side tabs are arranged such that the distal sides 53 and 63 of the proximal-side tabs 5 and 6 are disposed in parallel with the proximal sides 34 and 44 of the distal-side tabs 3 and 4. Therefore, if the proximal sides 34 and 44 of the distal-side tabs 3 and 4 are linear, the distal sides 53 and 63 of the proximal-side tabs 5 and 6 are also linear. If the proximal sides 34 and 44 of the distal-side tabs 3 and 4 are curved, the distal sides 53 and 63 of the proximal-side tabs 5 and 6 are also curved. The proximal sides 34 and 44 of the distal-side tabs 3 and 4 and the distal sides 53 and 63 of the proximal-side tabs 5 and 6 are preferably linear because the positional relationship between the proximal sides 34 and 44 of the distal-side tabs 3 and 4 and the distal sides 53 and 63 of the proximal-side tabs 5 and 6 is easy to be adjusted.

The proximal-side tabs 5 and 6 and the distal-side tabs 3 and 4 are formed such that the distal sides 53 and 63 of the proximal-side tabs 5 and 6 and the proximal sides 34 and 44 of the distal-side tabs 3 and 4 are disposed in parallel with each other with a distance larger than the diameter of the flexible medical component 7 to be held. Specifically, a distance L1 between the distal side 53 of the proximal-side tab 5 and the proximal side 34 of the distal-side tab 3 and a distance L2 between the distal side 63 of the proximal-side tab 6 and the proximal side 44 of the distal-side tab 4 are set larger than the diameter of the flexible medical component 7 to be held.

The tips 52 and 62 of the proximal-side tabs 5 and 6 may have V-shapes or smoothly curved U-shapes. The tip of V-shape is more preferable. The V-shapes of the tips 52 and 62 can provide a long length from the base 51 and 61 to the tip 52 and 62, thereby it becomes easier to hold the flexible medical component 7. The V-shaped tips 52 and 62 preferably have slightly rounded corners to prevent the tips 52 and 62 from sticking in an operator's hand and prevent the tips 52 and 62 from damaging, for example, the film of a package when the flexible medical component 7 is held and packaged.

The proximal-side tabs 5 and 6 have proximal sides 54 and 64 , and the proximal sides 54 and 64 may be linear or smoothly curved. In order to improve the holding force of the proximal-side tabs 5 and 6, that is, the force for holding the flexible medical component 7, the proximal-side tabs 5 and 6 preferably have large surface areas and have the long tab bases 51 and 61. The proximal sides 54 and 64 of the proximal-side tabs 5 and 6 are preferably linear because the lengths of the tab bases 51 and 61 can be secured while keeping the large surface areas of the proximal-side tabs 5 and 6.

Thus, the proximal-side tabs 5 and 6 preferably include the linear distal sides 53 and 63, the linear proximal sides 54 and 64, and the V-shaped tips 52 and 62.

The proximal-side tabs 5 and 6 formed thus can reliably hold the flexible medical component 7. Moreover, when the flexible medical component 7 having the curved distal portion 71 is held on the mounting card 1 and then is got out from the mounting card 1, the distal portion 71 of the flexible medical component 7 is not caught by the proximal-side tabs 5 and 6, preventing the catheter 7 from being deformed or broken by the distal-side tabs 3 and 4.

The flat surface 2 may optionally have tabs having other shapes in addition to the distal-side tabs 3 and 4 and the proximal-side tabs 5 and 6.

For example, the flat surface 2 may include a tunnel-like tab 8 that has a through hole for holding a bulky connector 72 typically attached to the proximal end side of the flexible medical component 7, and a positioning tab 9 that suppress a displacement of the holding position of the flexible medical component 7 on the flat surface 2.

The flexible medical component 7 may be a catheter, a sheath, and so on. The distal portion 71 of the flexible medical component 7 may be curved. The mounting card 1 of the present invention is suitable for holding catheters typically provided with curved distal portion.

A method of using the mounting card 1 will be described below. The elongated flexible medical component 7 is held by the tunnel-like tab 8 and the positioning tab 9 that are optionally provided; meanwhile, the distal-side tabs 3 and 4 and the proximal-side tabs 5 and 6 are lifted to hold the flexible medical component 7 between the flat surface 2 and the distal-side tabs 3 and 4 and the proximal-side tabs 5 and 6. The flexible medical component 7 is held thus on the flat surface 2 of the mounting card 1.

The distal-side tabs 3 and 4 and the proximal-side tabs 5 and 6 are optional and thus there is no need to use all of them.

For example, if the flexible medical component 7 having the curved distal portion 71 is held, tabs faced toward the same direction as the curving direction of the distal portion 71 are selected from among the distal-side tabs 3 and 4 and are used. In FIG. 3, the flexible medical component 7 includes the distal portion 71 that is curved downward. If the flexible medical component 7 like this is held, the distal-side tabs 3 which is faced toward downward same as the curving direction of the distal portion 71 in FIG. 3 are selected and are used. In other words, the flexible medical component 7 having the distal portion 71 that is curved downward is held by the pairs of the distal-side tabs 3 and the proximal-side tabs 5. In this case, the pairs of the distal-side tabs 4 and the proximal-side tabs 6 are unused and constitute the flat surface 2 without being lifted.

After the flexible medical component 7 is held on the mounting card 1, the mounting card with the flexible medical component 7 is packaged into a packaging bag according to a conventional method. After that, the flexible medical component 7 is optionally sterilized.

The package obtained thus is opened by, for example, a doctor to get out the flexible medical component 7 from the package for the use of the flexible medical component 7. When the flexible medical component 7 is got out, typically, the mounting card 1 with the flexible medical component 7 is got out from the packaging bag and then the flexible medical component 7 is carefully detached from the mounting card 1. However, since the flexible medical component 7 is long, it tends to open the packaging bag from the proximal end side of the flexible medical component 7, hold the proximal end side of the flexible medical component 7 and pull out the flexible medical component 7 from the packaging bag and the mounting card 1. As the mounting card 1 according to the present invention has the above-described configuration, the flexible medical component is not deformed or broken even if the flexible medical component 7 is pulled out from the mounting card 1.

### [Reference Signs List]

- 1: mounting card
- 2: flat surface
- 3, 4: distal-side tab
- 5, 6: proximal-side tab
- 31, 41, 51, 61: tab base
- 311, 411: distal end of tab base
- 32, 42, 52, 62: tip
- 33, 43, 53, 63: distal side
- 34, 44, 54, 64: proximal side
- 7: flexible medical component
- 71: distal portion
- 8: tunnel-like tab
- 9: positioning tab

## Claims

1. A mounting card (1) having a flat surface (2), the mounting card comprising an elongated flexible medical component (7) held on the mounting card, wherein
the flat surface (2) comprises a distal-side tab (3,4) and a proximal-side tab (5,6) that are formed in a pair so as to hold the flexible medical component (7) on the flat surface (2), and
the distal-side tab (3,4) and the proximal-side tab (5,6) are formed in a pair such that the distal-side tab (3,4) is located closer to a distal end of the flexible medical component (7) than the proximal-side tab (5,6) when the flexible medical component (7) is held on the mounting card (1),
the distal-side tab (3,4) is formed by making a cut on the mounting card (1), the distal-side tab (3,4) comprising a distal side (33,43) and a proximal side (34,44) and
the proximal-side tab (5,6) is formed by making a cut on the mounting card (1), the proximal-side tab (5,6) comprising a distal side (53, 63) and a proximal side (54,64), wherein the distal side (53, 63) of the proximal-side tab is disposed in parallel with the proximal side (34, 44) of the distal-side tab **characterized in that** the distal-side tab (3,4) is formed by making a U-shaped cut on the mounting card (1) such that a tip (32,42) of the distal-side tab (3,4) is oriented toward the distal end side of the flexible medical component (7),and
the proximal-side tab (5,6) is formed by making a cut on the mounting card (1) such that a distance between the distal side (53,63) of the cut of the proximal-side tab and the proximal side (34, 44) of the U-shaped cut of the distal-side tab is larger than a diameter of the flexible medical component (7).

2. The mounting card (1) according to claim 1, wherein the proximal-side tab (5,6) is formed by making a V-shaped cut on the mounting card (1).

## Patentansprüche

1. Befestigungskarte (1), die eine flache Oberfläche (2) aufweist und eine auf der Befestigungskarte gehaltene längliche flexible medizinische Komponente (7) umfasst, wobei
die flache Oberfläche (2) eine distale Lasche (3,4) und eine proximale Lasche (5, 6) aufweist, die paarweise ausgebildet sind, um die flexible medizinische Komponente (7) auf der flachen Oberfläche (2) zu halten, und
die distale Lasche (3,4) und die proximale Lasche (5,6) paarweise so ausgebildet sind, dass die distale Lasche (3,4) näher an dem distalen Ende der flexiblen medizinischen Komponente (7) liegt als die proximale Lasche (5,6), wenn die flexible medizinische Komponente (7) auf der Befestigungskarte (1) gehalten ist,
die durch einen Schnitt in der Befestigungskarte (1) gestaltete distale Lasche (3,4) eine distale Seite (33,43) und eine proximale Seite (34,44) hat, und
die durch einen Schnitt in der Befestigungskarte (1) gestaltete proximale Lasche (5,6) eine distal Seite (53,63) und eine proximale Seite (54,64) hat, wobei die distale Seite (53,63) der proximalen Lasche parallel zur proximalen Seite (34,44) der distalen Lasche angeordnet ist, **dadurch gekennzeichnet, dass**
die distale Lasche (3,4) durch einen U-förmigen Schnitt in der Befestigungskarte (1) so gestaltet ist, dass die Spitze (32,42) der distalen Lasche (3,4) zum distalen Ende der flexiblen medizinischen Komponente (7) hin ausgerichtet ist, und
die proximale Lasche (5,6) durch einen Schnitt in der Befestigungskarte (1) so gestaltet ist, dass der Abstand zwischen der distalen Seite (53, 63) des Schnitts der proximalen Lasche und der proximalen Seite (34,44) des U-förmigen Schnitts der distalen Lasche größer ist als der Durchmesser der flexiblen medizinischen Komponente (7).

2. Befestigungskarte (1) nach Anspruch 1, wobei die proximale Lasche (5,6) durch einen V-förmigen Schnitt in der Befestigungskarte (1) gestaltet ist.

## Revendications

1. Carte de montage (1) ayant une surface plate (2), la carte de montage comprenant un composant médical flexible allongé (7) maintenu sur la carte de montage, dans laquelle la surface plate (2) comprend une patte côté distal (3, 4) et une patte côté proximal (5, 6) qui sont formées en une paire de manière à maintenir le composant médical flexible (7) sur la surface plate (2), et
la patte côté distal (3, 4) et la patte côté proximal (5, 6) sont formées en une paire de telle sorte que la patte côté distal (3, 4) se situe plus près d'une extrémité distale du composant médical flexible (7) que la patte côté proximal (5, 6) quand le composant médical flexible (7) est maintenu sur la carte de montage (1),
la patte côté distal (3, 4) est formée en créant une découpe sur la carte de montage (1), la patte côté distal (3, 4) comprenant un côté distal (33, 43) et un côté proximal (34, 44), et
la patte côté proximal (5, 6) est formée en créant une découpe sur la carte de montage (1), la patte côté proximal (5, 6) comprenant un côté distal (53, 63) et un côté proximal (54, 64), dans laquelle le côté distal (53, 63) de la patte côté proximal est disposé parallèlement au côté proximal (34, 44) de la patte côté distal, **caractérisée en ce que** la patte côté distal (3, 4) est formée en créant une découpe en forme de U sur la carte de montage (1) de telle sorte qu'une pointe (32, 42) de la patte côté distal (3, 4) est orientée vers le côté d'extrémité distale du composant médical flexible (7), et
la patte côté proximal (5, 6) est formée en créant une découpe sur la carte de montage (1) de telle sorte qu'une distance entre le côté distal (53, 63) de la découpe de la patte côté proximal et le côté proximal (34, 44) de la découpe en forme de U de la patte côté distal est supérieure à un diamètre du composant médical flexible (7).

2. Carte de montage (1) selon la revendication 1, dans laquelle la patte côté proximal (5, 6) est formée en créant une découpe en forme de V sur la carte de montage (1).
